(19) Europäisches Patentamt
European Patent Office
Office européen des brevets

(11) Publication number: **0 243 467 B2**

(12) # NEW EUROPEAN PATENT SPECIFICATION

(45) Date of publication of the new patent specification: **14.09.94**

(51) Int. Cl.[5]: **C07D 207/404**, C07F 1/00, C10M 133/16, C10L 1/22, //C10N10/00,C10N40/00

(21) Application number: **86906642.3**

(22) Date of filing: **17.10.86**

(86) International application number: **PCT/US86/02207**

(87) International publication number: **WO 87/02663 (07.05.87 87/10)**

(54) **COMPOSITIONS, CONCENTRATES, LUBRICANT COMPOSITIONS, FUEL COMPOSITION AND METHODS FOR IMPROVING FUEL ECONOMY OF INTERNAL COMBUSTION ENGINES.**

(30) Priority: **25.10.85 US 791260**

(43) Date of publication of application: **04.11.87 Bulletin 87/45**

(45) Publication of the grant of the patent: **19.08.92 Bulletin 92/34**

(45) Mention of the opposition decision: **14.09.94 Bulletin 94/37**

(84) Designated Contracting States: **AT BE CH DE FR GB IT LI LU NL SE**

(56) References cited:
**EP-A- 0 020 037**
**EP-A- 0 074 199**
**GB-A- 1 054 280**
**GB-A- 1 575 467**
**US-A- 2 628 942**
**US-A- 3 018 291**
**US-A- 3 068 082**
**US-A- 4 325 827**

**Houben-Weyl, Methoden der Organischen Chemie, volume XIII/I page 449 and volume VIII, page 657**

(73) Proprietor: **The Lubrizol Corporation**
**29400 Lakeland Boulevard**
**Wickliffe, Ohio 44092 (US)**

(72) Inventor: **WALSH, Reed, H.**
**8785 Springvalley Drive**
**Mentor, OH 44060 (US)**

(74) Representative: **Crisp, David Norman et al**
**D. YOUNG & CO.**
**21 New Fetter Lane**
**London EC4A 1DA (GB)**

EP 0 243 467 B2

## Description

This invention relates to compositions useful as lubricant and fuel additives and methods for improving the operation of internal combustion engines, specifically by reducing the amount of fuel consumed by such engines. More particularly, the invention includes lubricating compositions which may be used in such engines to decrease fuel consumption, and a method of using such lubricating compositions to accomplish this purpose. This invention also relates to a method for preparing these compositions. Additionally, this invention relates to concentrates and fuel composition comprising these compositions.

Efforts to reduce the amount of fuel consumed by internal combustion engines such as automobile engines have increased in recent years as a result of the petroleum shortage, the increased cost of petroleum products, and the desire for conservation of natural resources such as petroleum. It is recognized that a situation under which fuel consumption is minimized is desirable, both because of the conservation factor and because such a situation is economical for the user of the engine.

Many of the proposed solutions to the fuel conservation problem have been mechanical as, for example, adjusting the engine for a leaner burn or simply building smaller cars and smaller engines. Other efforts have related to developing lubricants that reduce the overall friction of the engine thereby reducing energy requirements. Some synthetic lubricants have been developed and compounded for use in the automobile engine to reduce fuel consumption. A considerable amount of effort has been expended toward developing additives for use in mineral lubricating oils and greases to reduce the friction properties of the oils and greases.

The following publications are exemplary of art in this area:

U.S. Patent 3,796,663 describes N-hydroxy hydrocarbyl-substituted cyclic imides of $C_4$-$C_5$ dicarboxylic acids, e.g., hydrocarbyl-substituted succinyl and glutaric hydroximides, as rust inhibition, wear reduction and frictional control additives for lubricating oils.

U.S. Patent 4,104,182 describes a lubricating oil composition comprising a hydrocarbyl oil of lubricating viscosity, a metal-containing additive characterized by promoting the formation of hard deposits in an internal combustion engine, and a hydrocarbon-substituted succinimide represented by the formula:

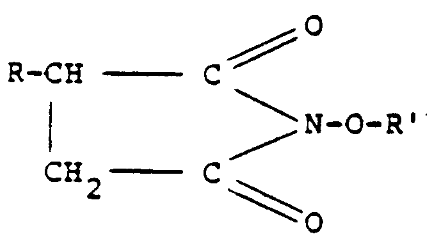

in which R is an aliphatic hydrocarbon radical having from about 1 to 50 carbon atoms and R' is a hydrocarbon radical having from 3 to 20 carbon atoms, and a method for lubricating an internal combustion engine.

U.S. Patent 4,325,827 describes a fuel efficient motor oil which contains a friction-reducing amount of an N-hydroxymethyl $C_{12-36}$ aliphatic hydrocarbyl succinimide. This patent also describes these additives as being used in liquid hydrocarbon engine fuel.

European Patent Application 20,037 describes oil soluble $C_{12-36}$ aliphatic hydrocarbyl succinimide or succinamide which provides a friction reducing effect where it is incorporated in a lubricating oil.

In one aspect, the invention provides a composition comprising one or more substituted succinimide derivatives represented by the formula:

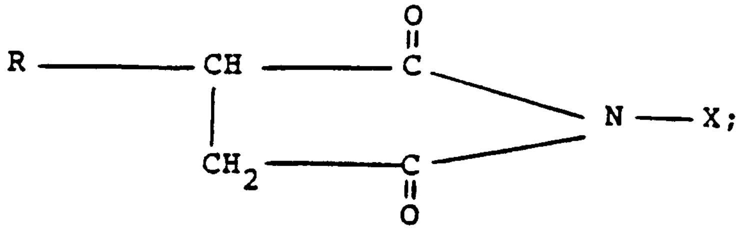

wherein R is a hydrocarbon-based group containing from 8 up to 35 carbon atoms, and X is magnesium, calcium, barium or zinc.

The invention also relates to the use of such compositions in lubricating oils and normally liquid fuels. Lubricating oils containing the compositions of the invention are effective in reducing the amount of fuel consumed by internal combustion engines. The invention also relates to a method of reducing the amount of fuel consumed by an internal combustion engine. In addition, lubricating oils containing the compositions of this invention are effective deposit softeners in internal combustion engines. This invention also relates to methods for preparing these substituted succinimide derivatives which are discussed hereinafter.

Various preferred features and embodiments of the invention are described below.

Generally, in the substituted succinimide derivatives of this invention, R is a hydrocarbon-based group containing from about 8 up to about 35 carbon atoms.

As used herein, the term "hydrocarbon-based group" denotes a radical having a carbon atom directly attached to the remainder of the molecule and having predominantly hydrocarbon character within the context of this invention. Such groups include the following:

(1) Hydrocarbon groups; that is, aliphatic (e.g., alkyl or alkenyl), alicyclic (e.g., cycloalkyl or cycloalkenyl), aromatic, aliphatic- and alicyclic-substituted aromatic, aromatic-substituted aliphatic and alicyclic radicals, and the like, as well as cyclic radicals wherein the ring is completed through another portion of the molecule (that is, any two indicated substituents may together form an alicyclic radical). Such groups are known to those skilled in the art.

(2) Substituted hydrocarbon groups; that is, radicals containing nonhydrocarbon substituents which, in the context of this invention, do not alter the predominantly hydrocarbon character of the radical. Those skilled in the art will be aware of suitable substituents; examples are halo, alkoxy, hydroxy, alkylthio, carbalkoxy, nitro and carboxyl.

(3) Hetero groups; that is, radicals which, while predominantly hydrocarbon in character within the context of this invention, contain atoms other than carbon present in a chain or ring otherwise composed of carbon atoms.

Suitable hetero atoms will be apparent to those skilled in the art and include, for example, nitrogen, oxygen and sulfur.

In general, no more than about three substituents or hetero atoms, and preferably no more than one, will be present for each 10 carbon atoms in the hydrocarbon-based group.

Terms such as "alkyl hydrocarbon-based group," "aliphatic hydrocarbon-based group," "aryl hydrocarbonbased group" and the like have meanings analogous to the above with respect to alkyl, aliphatic and aryl groups and the like.

Preferably, the hydrocarbon-based groups in the compositions of this invention are free from acetylenic unsaturation.

As used in the present specification and claims, the term "lower," when used in conjunction with terminology designating a chemical group such as alkyl, alkenyl, alkylene and the like, is intended to describe such groups having a total carbon atom content of up to and including 7. For example, "lower alkyl" includes all straight and branched chain alkyl groups of up to and including 7 carbon atoms.

Typically the substituent groups are aliphatic hydrocarbon-based groups containing from about 8 up to about 35 carbon atoms, preferably from about 10 up to about 30 carbon atoms and more preferably from about 12 up to about 28 carbon atoms. The R group may be a branched chain or straight chain configuration; however, it is preferred that at least 8 carbon atoms are in a straight chain configuration and, more preferably, is substantially straight chain configuration. Furthermore, the R group is preferably alkyl or alkenyl.

The term "substantially straight-chain" means that the group contains no more than about 2 methyl groups.

As used herein, X is magnesium, calcium, barium or zinc. Preferably, X is selected from calcium, barium and zinc. The substituted succinimide derivatives of this invention may be metal complexes, metal salts or mixtures thereof. The metal salts formed can be "acidic," "neutral" or "basic" salts. An "acidic" salt is one in which the equivalents of succinimide exceed the stoichiometric amounts required to neutralize the number of equivalents of metal. A "neutral" salt is one wherein the metal and succinimide are present in stoichiometrically equivalent amounts. A "basic" or "overbased" salt (sometimes referred to as "superbased" or "hyperbased" salts) is one wherein the metal is present in a stoichiometric excess relative to the number of stoichiometric equivalents of substituted succinimide from which it is produced.

The substituted succinimide metal derivatives of the instant invention include coordination compounds. Such compounds being a complex of the substituted succinimide and the metal cation. Coordination compounds are well known to those of ordinary skill and are described in detail in "Advanced Inorganic

wherein R is an aliphatic hydrocarbon-based group, free from acetylenic unsaturation, containing from 10 up to 30 carbon atoms, in which at least 8 carbon atoms are in a straight-chain configuration and X is magnesium, calcium, barium or zinc.

The invention also relates to the use of such compositions in lubricating oils and normally liquid fuels. Lubricating oils containing the compositions of the invention are effective in reducing the amount of fuel consumed by internal combustion engines. The invention also relates to a method of reducing the amount of fuel consumed by an internal combustion engine. In addition, lubricating oils containing the compositions of this invention are effective deposit softeners in internal combustion engines. This invention also relates to methods for preparing these substituted succinimide derivatives which are discussed hereinafter.

Various preferred features and embodiments of the invention are described below.

As used herein, the term hydrocarbon-based group" denotes a radical having a carbon atom directly attached to the remainder of the molecule and having predominantly hydrocarbon character within the context of this invention. Such groups include the following:

(1) Hydrocarbon groups; that is, aliphatic (e.g., alkyl or alkenyl), alicyclic (e.g., cycloalkyl or cycloalkenyl), aromatic, aliphatic- and alicyclic-substituted aromatic, aromatic-substituted aliphatic and alicyclic radicals, and the like, as well as cyclic radicals wherein the ring is completed through another portion of the molecule (that is, any two indicated substituents may together form an alicyclic radical). Such groups are known to those skilled in the art.

(2) Substituted hydrocarbon groups; that is, radicals containing nonhydrocarbon substituents which, in the context of this invention, do not alter the predominantly hydrocarbon character of the radical. Those skilled in the art will be aware of suitable substituents; examples are halo, alkoxy, hydroxy, alkylthio, carbalkoxy, nitro and carboxyl.

(3) Hetero groups; that is, radicals which, while predominantly hydrocarbon in character within the context of this invention, contain atoms other than carbon present in a chain or ring otherwise composed of carbon atoms.

Suitable hetero atoms will be apparent to those skilled in the art and include, for example, nitrogen, oxygen and sulfur.

In general, no more than about three substituents or hetero atoms, and preferably no more than one, will be present for each 10 carbon atoms in the hydrocarbon-based group.

Terms such as "alkyl hydrocarbon-based group," "aliphatic hydrocarbon-based group," "aryl hydrocarbonbased group" and the like have meanings analogous to the above with respect to alkyl, aliphatic and aryl groups and the like.

Preferably, the hydrocarbon-based groups in the compositions of this invention are free from acetylenic unsaturation.

As used in the present specification and claims, the term "lower," when used in conjunction with terminology designating a chemical group such as alkyl, alkenyl, alkylene and the like, is intended to describe such groups having a total carbon atom content of up to and including 7. For example, "lower alkyl" includes all straight and branched chain alkyl groups of up to and including 7 carbon atoms.

The substituent groups are aliphatic hydrocarbon-based groups, free from acetylenic unsaturation, containing from about 10 up to about 20 carbon atoms, preferably from about 12 up to about 28 carbon atoms. The R group may be a branched chain or straight chain configuration; however, at least 8 carbon atoms are in a straight chain configuration and, more preferably, is substantially straight chain configuration. Furthermore, the R group is preferably alkyl or alkenyl.

The term "substantially straight-chain" means that the group contains no more than about 2 methyl groups.

As used herein, X is magnesium, calcium, barium or zinc. Preferably, X is selected from calcium, barium and zinc. The substituted succinimide derivatives of this invention may be metal complexes, metal salts or mixtures thereof. The metal salts formed can be "acidic," "neutral" or "basic" salts. An "acidic" salt is one in which the equivalents of succinimide exceed the stoichiometric amounts required to neutralize the number of equivalents of metal. A "neutral" salt is one wherein the metal and succinimide are present in stoichiometrically equivalent amounts. A "basic" or "overbased" salt (sometimes referred to as "superbased" or "hyperbased" salts) is one wherein the metal is present in a stoichiometric excess relative to the number of stoichiometric equivalents of substituted succinimide from which it is produced.

The substituted succinimide metal derivatives of the instant invention include coordination compounds. Such compounds being a complex of the substituted succinimide and the metal cation. Coordination compounds are well known to those of ordinary skill and are described in detail in "Advanced Inorganic using the invention for its intended purposes. "Substantially inert" as used herein is, thus, readily understood and appreciated by those of ordinary skill in the art.

As used in the specification and the appended claims, the term "solvent/diluent medium" is intended to include those solvent/diluent media in which independently each of the reactants are soluble or stably dispersible. The term "stably dispersible" as used in the specification and the appended claims is intended to mean a composition (e.g., a single compound, a mixture of two or more compounds, etc.) is capable of being dispersed in a given medium to an extent which allows it to function in its intended manner. Thus, for example, where a composition is prepared by a reaction in an oil, it is sufficient that the reactants be capable of being suspended in the oil in a manner sufficient to allow the reaction to occur and the formation of the composition. Thus, the term "solvent/diluent medium" is understood and can be used in a conventional manner by those of ordinary skill in the art.

The compositions of this invention may be used as a lubricant additive. However, the compositions sometimes may be accompanied by the formation of by-products and/or excess solvent/diluent medium which may lessen its commercial appeal. Accordingly, these undesirable by-product and/or excess of undesired solvent/diluent medium can be separated from the compositions of this invention by techniques known in the art; e.g., filtration, evaporation (e.g., stripping), etc., to obtain a more desirable product. Alternatively, if the solvent/diluent medium is, for example, a lubricant base suitable for use in the lubricating compositions of this invention, the product can be left in the solvent/diluent medium and used to form the lubricating compositions as described below.

This invention is exemplified in the following examples. Of course, these examples are not intended as limiting this invention as modification of the examples by ordinary expedient will be readily apparent to those of ordinary skill in the art.

In all examples, unless otherwise stated, all parts are parts by weight and all percentages are derived from parts by weight.

Example 1

5775 parts (25.33 moles) of a commercially available $C_{15-18}$ alpha-olefin fraction (having a carbon number distribution of 1% $C_{14}$, 29% $C_{15}$, 28% $C_{16}$, 27% $C_{17}$, 14% $C_{18}$, and 1% $C_{19}$) are passed through a 300 mm (12-inch) column packed with activated alumina into a 12-liter flask containing 2485 parts (25.35 moles) maleic anhydride. The mixture is heated to 214°C and maintained at that temperature for 7 hours with a nitrogen sparge ($6 \times 10^{-3} m^3$ (0.2 standard cubic feet) per hour) and then cooled to room temperature. The mixture is then heated to 209°-212°C and maintained at that temperature for 7 hours, then cooled to room temperature. 1500 parts of textile spirits are added and the mixture is stirred for one hour. The mixture is filtered with diatomaceous earth. The mixture is stripped under a vacuum of 90 Pa (0.7 mm Hg) at 168°C then cooled to room temperature. The mixture is filtered with diatomaceous earth at room temperature. The filtrate is the desired product.

Example 2

A reaction vessel containing 2528 parts (8 moles) of the $C_{15-18}$ substituted succinic anhydride of Example 1 is heated to 100°C. Gaseous ammonia, 252 parts (14.8 moles), is slowly bubbled under the surface over two hours during which the temperature of the reaction mixture is increased to 280°C. Water of reaction is removed continuously during the ammonia addition. The reaction mixture is stripped at 180°C under a vacuum of 2 kPa (15 mm Hg). The residue is a $C_{15-18}$ substituted succinimide having an acid number to phenolphthalein of 80 and a percent nitrogen of 4.81.

Example 3

A mixture of 1264 parts (4 moles) of the $C_{15-18}$ substituted succinic anhydride of Example 1,240 parts (4 moles) urea and 200 parts xylene is heated to reflux and water of reaction is removed by azeotropic distillation. The volatiles are removed at 160°C and 3kPa (22 mm Hg). The reaction mixture is filtered through a filter aid material to yield the desired $C_{15-18}$ succinimide with an acid number to phenolphthalein of 69.5.

Example 4

Charged to a reaction vessel are 198 parts (2.02 moles) of maleic anhydride and 500 parts (1.36 moles) of a commercial mixture of $C_{18-24}$ olefins available from Ethyl Corporation wherein these olefins are typically 10% $C_{18}$, 45% $C_{20}$, 25% $C_{22}$ and 15% $C_{24}$ and are comprised predominantly of substantially

straight chain alpha, 1,1 -disubstituted and 1,2-disubstituted olefins. This reaction mixture is heated to 200 °C and held at 200-220 °C for 10 hours. Unreacted starting materials are removed by vacuum distillation to 700 Pa (5 mm Hg) at 200 °C. The reaction mixture is filtered to yield the desired $C_{18-24}$ substituted succinic anhydride having an acid number of 290.

Example 5

A reaction vessel containing 5424 parts (12 moles) of the $C_{18-24}$ substituted succinic anhydride of Example 4 is heated to 120 °C. Gaseous ammonia, 287 parts (15.7 moles), is slowly bubbled below the surface over 6.75 hours during which the temperature of the reaction mixture is increased to 240 °C. Water of reaction is removed continuously during the $NH_3$ addition. The reaction mixture is stripped to 190 °C. The residue is the $C_{18-24}$ substituted succinimide having an acid number to phenolphthalein of 69 and a per cent nitrogen of 2.91.

Example 6

A reactor is charged with 304 parts (4 moles) of 2-methoxyethanol and is heated to 120 °C. Magenesium metal is charged in 5 increments with each increment being reacted before charging the next increment. A total of 24 parts (1 mole) of magnesium is added in this manner. At 140 °C, a mixture of 886 parts (2 moles) of the $C_{18-24}$ substituted succinimide of Example 5 is diluted with 400 parts xylene. The temperature is held at 140 °C for 5 hour and the volatiles are removed by vacuum distillation to 2 kPa (15 mm Hg). The reaction mixture is filtered through a filter aid material to give the desired product.

Example 7

Charged to a reactor are 483 parts (4.72 equivalents) of maleic anhydride and 1000 parts (5.95 equivalents) of polypropylene tetramer. This reaction mixture is heated to 182 °C and held at this temperature for 9 hours. Unreacted starting materials are removed by vacuum distillation at 190 °C and 1.3 kPa (10 mm Hg). The reaction mixture is filtered to yield the desired polypropylene tetramer-substituted succinic anhydride having an acid number to phenolphthalein of 428.

Example 8

A reaction vessel containing 2388 parts (8 moles) of the polypropylene tetramer substituted succinic anhydride of Example 7 is heated to 120 °C. Gaseous ammonia, 136 parts (8 moles), is slowly bubbled below the surface over 7.5 hours during which the temperature of the reaction mixture is increased to 155 °C. Water of reaction is removed continuously during the $NH_3$ addition. The reaction mixture is filtered through a filter aid material to yield the polypropylene tetramer substituted succinimide having a 5.18% nitrogen content and an acid number to phenolphthalein of 44.

Example 9

A mixture of 442.5 parts (1.5 moles) of the polypropylene tetramer substituted succinimide of Example 8 84 parts of (1.5 moles) potassium hydroxide and 250 parts toluene are heated to reflux for 2 hours and water is removed by azeotropic distillation. This reaction mixture is cooled to 90 ° C and 102 parts (0.75 moles) of zinc chloride is added and the temperature is increased to reflux and held for 7 hours. Diluent oil, 186 parts, is added at 90 °C and the volatiles are removed by stripping at 120 °C under 4-5 kPa (30-40 mm Hg) vacuum. The reaction mixture is filtered through a filter aid material to give the desired product having a 3.2% nitrogen content and 7.49% zinc content.

Example 10

A mixture of 620 parts (1.5 moles) of the $C_{18-24}$ substituted succinimide of Example 5, 500 parts xylene and 675 parts duluent oil is heated to 80 °C and 50 parts water and 55.5 parts (0.75 moles) calcium hydroxide are added. The reaction mixture is stripped to 180 °C under 1.6 kPa (12 mm Hg) vacuum. The reaction mixture is filtered through a filter aid material to yield the desired product having a 1.96% calcium content and a 1.55% nitrogen content.

As previously indicated, the compositions of this invention are also useful as additives for lubricants, in which they can function primarily as friction modifiers and/or deposit softeners. They can be employed in a variety of lubricants based on diverse oils of lubricating viscosity, including natural and synthetic lubricating oils and mixtures thereof. These lubricants include crankcase lubricating oils for spark-ignited and compression-ignited internal combustion engines, including automobile and truck engines, two-cycle engines, aviation piston engines, marine and railroad diesel engines, and the like. They can also be used in gas engines, stationary power engines and turbines and the like. Automatic transmission fluids, transaxle lubricants, gear lubricants, metal-working lubricants, hydraulic fluids and other lubricating oil and grease compositions can also benefit from the incorporation therein of the compositions of the present invention.

Natural oils include animal oils and vegetable oils (e.g., castor, lard oil) liquid petroleum oils and hydrorefined, solvent-treated or acid-treated mineral lubricating oils of the paraffinic, naphthenic and mixed paraffinic-naphthenic types. Oils of lubricating viscosity derived from coal or shale are also useful base oils.

Synthetic lubricating oils include hydrocarbon oils and halo-substituted hydrocarbon oils such as polymerized and interpolymerized olefins (e.g., polybutylenes, polypropylenes, propylene-isobutylene copolymers, chlorinated polybutylenes, poly(1-hexenes), poly(1-octenes), poly(1-decenes)); alkylbenzenes (e.g., dodecylbenzenes, tetradecylbenzenes, dinonylbenzenes, di(2-ethylhexyl)benzenes); polyphenyls (e.g., biphenyls, terphenyls, alkylated polyphenols); and alkylated diphenyl ethers and alkylated diphenyl sulfides and the derivatives, analogs and homologs thereof.

Alkylene oxide polymers and interpolymers and derivatives thereof where the terminal hydroxyl groups have been modified by esterification, etherification, etc., constitute another class of known synthetic lubricating oils. These are exemplified by polyoxyalkylene polymers prepared by polymerization of ethylene oxide or propylene oxide, the alkyl and aryl ethers of these polyoxyalkylene polymers (e.g., methylpolyisopropylene glycol ether having an average molecular weight of 1000, diphenyl ether of poly-ethylene glycol having a molecular weight of 500-1000, diethyl ether of polypropylene glycol having a molecular weight of 1000-1500); and mono- and polycarboxylic esters thereof, for example, the acetic acid esters, mixed $C_3$-$C_8$ fatty acid esters and $C_{13}$ Oxo acid diester of tetraethylene glycol.

Another suitable class of synthetic lubricating oils comprises the esters of dicarboxylic acids (e.g., phthalic acid, succinic acid, alkyl succinic acids and alkenyl succinic acids, maleic acid, azelaic acid, suberic acid, sebacic acid, fumaric acid, adipic acid, linoleic acid dimer, malonic acid, alkylmalonic acids, alkenyl malonic acids) with a variety of alcohols (e.g., butyl alcohol, hexyl alcohol, dodecyl alcohol, 2-ethylhexyl alcohol, ethylene glycol, diethylene glycol monoether, propylene glycol). Specific examples of these esters include dibutyl adipate, di(2-ethylhexyl) sebacate, di-n-hexyl fumarate, dioctyl sebacate, diisooctyl azelate, diisodecyl azelate, dioctyl phthalate, didecyl phthalate, dieicosyl sebacate, the 2-ethylhexyl diester of linoleic acid dimer, and the complex ester formed by reacting one mole of sebacic acid with two moles of tetraethylene glycol and two moles of 2-ethylhexanoic acid.

Esters useful as synthetic oils also include those made from $C_5$ to $C_{12}$ monocarboxylic acids and polyols and polyol ethers such as neopentyl glycol, trimethylolpropane, pentaerythritol, dipentaerythritol and tripentaerythritol, etc.

Silicon-based oils such as the polyalkyl-, polyaryl-, polyalkoxy-, or polyaryoxysiloxane oils and silicate oils comprise another useful class of synthetic lubricants; they include tetraethyl silicate, tetraisopropyl silicate, tetra-(2-ethylhexyl) silicate, tetra-(4-methyl-2-ethyl-hexyl) silicate, tetra-(p-tert-butylphenyl) silicate, hexa-(4-methyl-2-pentoxy)disiloxane, poly(methyl)siloxanes and poly(methylphenyl) siloxanes. Other synthetic lubricating oils include liquid esters of phosphorus-containing acids (e.g., tricresyl phosphate, trioctyl phosphate, diethyl ester of decylphosphonic acid) and polymeric tetrahydrofurans.

Unrefined, refined and rerefined oils can be used in the lubricants of the present invention. Unrefined oils are those obtained directly from a natural or synthetic source without further purification treatment. For example, a shale oil obtained directly from retorting operations, a petroleum oil obtained directly from distillation or ester oil obtained directly from an esterification process and used without further treatment would be an unrefined oil. Refined oils are similar to the unrefined oils except they have been further treated in one or more purification steps to improve one or more properties. Many such purification techniques, such as distillation, solvent extraction, acid or base extraction, filtration and percolation are known to those skilled in the art. Rerefined oils are obtained by processes similar to those used to obtain refined oils applied to refined oils which have been already used in service. Such rerefined oils are also known as reclaimed or reprocessed oils and often are additionally processed by techniques for removal of spent additives and oil breakdown products.

Generally the lubricants of the present invention contain an amount of the composition of this invention sufficient to provide it with friction modification and/or deposit softening properties. Normally, this amount employed will be about 0.05 percent to about 20 percent, preferably about 0.1 percent to about 10 percent

of the total weight of the lubricating composition. This amount is exclusive of solvent/diluent medium. In lubricating compositions operated under extremely adverse conditions, such as lubricating compositions for marine diesel engines, the compositions of this invention may be present in amounts of up to about 30 percent by weight, or more, of the total weight of the lubricating composition.

The term "minor amount" as used in the specification and appended claims is intended to mean that when a composition contains a "minor amount" of a specific material that amount is less than 50 percent by weight of the composition.

The term "major amount" as used in the specification and appended claims is intended to mean that when a composition contains a "major amount" of a specific material that amount is more than 50 percent by weight of the composition.

The invention also contemplates the use of other additives in combination with the compositions of this invention. Such additives include, for example, detergents and dispersants of the ash-producing or ashless type, corrosion- and oxidation-inhibiting agents, pour point depressing agents, extreme pressure agents, antiwear agents, color stabilizers and anti-foam agents.

The ash-producing detergents are exemplified by oil-soluble neutral and basic salts of alkali or alkaline earth metals with sulfonic acids, carboxylic acids, or organic phosphorus acids characterized by at least one direct carbon-to-phosphorus linkage such as those prepared by the treatment of an olefin polymer (e.g., polyisobutene having a molecular weight of 1000) with a phosphorizing agent such as phosphorus trichloride, phosphorus heptasulfide, phosphorus pentasulfide, phosphorus trichloride and sulfur, white phosphorus and a sulfur halide, or phosphorothioic chloride. The most commonly used salts of such acids are those of sodium, potassium, lithium, calcium, magnesium, strontium and barium.

The term "basic salt" is used to designate metal salts wherein the metal is present in stoichiometrically larger amounts than the organic acid radical. The commonly employed methods for preparing the basic salts involve heating a mineral oil solution of an acid with a stoichiometric excess of a metal neutralizing agent such as the metal oxide, hydroxide, carbonate, bicarbonate, or sulfide at a temperature of at least about 50 °C and filtering the resulting mass. The use of a "promoter" in the neutralization step to aid the incorporation of a large excess of metal likewise is known. Examples of compound useful as the promoter include phenolic substances such as phenol, naphthol, alkylphenol, thiophenol, sulfurized alkylphenol, and condensation products of formaldehyde with a phenolic substance; alcohols such as methanol, 2-propanol, octyl alcohol, cellosolve, carbitol, ethylene glycol, stearyl alcohol, and cyclohexyl alcohol; and amines such as aniline, phenylenediamine, phenothiazine, phenyl-naphthylamine, and dodecylamine. A particularly effective method for preparing the basic salts comprises mixing an acid with an excess of a basic alkaline earth metal neutralizing agent and at least one alcohol promoter, and carbonating the mixture at an elevated temperature such as 60-200 °C.

Ashless detergents and dispersants are so called despite the fact that, depending on its constitution, the dispersant may upon combustion yield a nonvolatile material such as boric oxide or phosphorus pentoxide; however, it does not ordinarily contain metal and therefore does not yield a metal-containing ash on combustion. Many types are known in the art, and any of them are suitable for use in the lubricant compositions of this invention. The following are illustrative:

(1) Reaction products of carboxylic acids (or derivatives thereof) containing at least about 34 and preferably at least about 54 carbon atoms with nitrogen-containing compounds such as amine, organic hydroxy compounds such as phenols and alcohols, and/or basic inorganic materials. Examples of these "carboxylic dispersants" are described in British Patent 1,306,529 and in many U.S. patents including the following:

| | | |
|---|---|---|
| 3,163,603 | 3,351,552 | 3,541,012 |
| 3,184,474 | 3,381,022 | 3,543,678 |
| 3,215,707 | 3,399,141 | 3,542,680 |
| 3,219,666 | 3,415,750 | 3,567,637 |
| 3,271,310 | 3,433,744 | 3,574,101 |
| 3,272,746 | 3,444,170 | 3,576,743 |
| 3,281,357 | 3,448,048 | 3,630,904 |
| 3,306,908 | 3,448,049 | 3,632,510 |
| 3,311,558 | 3,451,933 | 3,632,511 |
| 3,316,177 | 3,454,607 | 3,697,428 |
| 3,340,281 | 3,467,668 | 3,725,441 |
| 3,341,542 | 3,501,405 | Re 26,433 |
| 3,346,493 | 3,522,197 | |

(2) Reaction products of relatively high molecular weight aliphatic or alicyclic halides with amines, preferably polyalkylene polyamines. These may be characterized as "amine dispersants" and examples thereof are described for example, in the following U.S. patents:

| | |
|---|---|
| 3,275,554 | 3,454,555 |
| 3,438,757 | 3,565,804 |

(3) Reaction products of alkyl phenols in which the alkyl group contains at least about 30 carbon atoms with aldehydes (especially formaldehyde) and amines (especially polyalkylene polyamines), which may be characterized as "Mannich dispersants". The materials described in the following U.S. patents are illustrative:

| | | |
|---|---|---|
| 2,459,112 | 3,442,808 | 3,591,598 |
| 2,962,442 | 3,448,047 | 3,600,372 |
| 2,984,550 | 3,454,497 | 3,634,515 |
| 3,036,003 | 3,459,661 | 3,649,229 |
| 3,166,516 | 3,461,172 | 3,697,574 |
| 3,236,770 | 3,493,520 | 3,725,277 |
| 3,355,270 | 3,539,633 | 3,725,480 |
| 3,368,972 | 3,558,743 | 3,726,882 |
| 3,413,347 | 3,586,629 | 3,980,569 |

(4) Products obtained by post-treating the carboxylic, amine or Mannich dispersants with such reagents as urea, thiourea, carbon disulfide, aldehydes, ketones, carboxylic acids, hydrocarbon-substituted succinic anhydrides, nitriles, epoxides, boron compounds, phosphorus compounds or the like. Exemplary materials of this kind are described in the following U.S. patents:

| | | | |
|---|---|---|---|
| 3,036,003 | 3,282,955 | 3,493,520 | 3,639,242 |
| 3,087,936 | 3,312,619 | 3,502,677 | 3,649,229 |
| 3,200,107 | 3,366,569 | 3,513,093 | 3,649,659 |
| 3,216,936 | 3,367,943 | 3,533,945 | 3,658,836 |
| 3,254,025 | 3,373,111 | 3,539,633 | 3,697,574 |
| 3,256,185 | 3,403,102 | 3,573,010 | 3,702,757 |
| 3,278,550 | 3,442,808 | 3,579,450 | 3,703,536 |
| 3,280,234 | 3,455,831 | 3,591,598 | 3,704,308 |
| 3,281,428 | 3,455,832 | 3,600,372 | 3,708,422 |

(5) Interpolymers of oil-solubilizing monomers such as decyl methacrylate, vinyl decyl ether and high molecular weight olefins with monomers containing polar substituents, e.g., aminoalkyl acrylates or acrylamides and poly-(oxyethylene)-subsituted acrylates. These may be characterized as "polymeric

dispersants" and examples thereof are disclosed in the following U.S. patents:

| | |
|---|---|
| 3,329,658 | 3,666,730 |
| 3,449,250 | 3,687,849 |
| 3,519,565 | 3,702,300 |

Extreme pressure agents and corrosion- and oxidation-inhibiting agents are exemplified by chlorinated aliphatic hydrocarbons such as chlorinated wax; organic sulfides and polysulfides such as benzyl disulfide, bis(chlorobenzyl)-disulfide, dibutyl tetrasulfide, sulfurized methyl ester of oleic acid, sulfurized alkylphenol, sulfurized dipentene, and sulfurized terpene; phosphosulfurized hydrocarbons such as the reaction product of a phosphorus sulfide with turpentine or methyl oleate, phosphorus esters including principally dihydrocarbon and trihydrocarbon phosphites such as dibutyl phosphite, diheptyl phosphite, dicyclohexyl phosphite, pentylphenyl phosphite, dipentylphenyl phosphite, tridecyl phosphite, distearyl phosphite, dimethyl napthyl phosphite, oleyl 4-pentylphenyl phosphite, polypropylene (molecular weight 500)-substituted phenyl phosphite, diisobutyl-substituted phenyl phosphite; metal thiocarbamates, such as zinc dioctyldithiocarbamate, and barium heptylphenyl dithiocarbamate; Group II metal phosphorodithioates such as zinc dicyclohexylphosphorodithioate, zinc dioctylphosphorodithioate, barium di(heptylphenyl)-phosphorodithioate, cadmium dinonylphosphorodithioate, and the zinc salt of a phosphorodithioic acid produced by the reaction of phosphorus pentasulfide with an equimolar mixture of isopropyl alcohol and n-hexyl alcohol.

The compositions of this invention can be added directly to the lubricant. Preferably, however, they are diluted with a substantially inert, normally liquid organic diluent such as mineral oil, naphtha, benzene, toluene or xylene, to form an additive concentrate. These concentrates usually contain from about 10 percent to 90 percent by weight of the composition of this invention and may contain, in addition, one or more other additives known in the art or described hereinabove.

The fuel compositions of the present invention contain a major proportion of a normally liquid fuel, usually a hydrocarbonaceous petroleum distillate fuel such as motor gasoline as defined by ASTM Specification D-439-73 and diesel fuel or fuel oil as defined by ASTM Specification D-396. Normally liquid fuel compositons comprising nonhydrocarbonaceous materials such as alcohols, ethers, organonitro compounds and the like (e.g., methanol, ethanol, diethyl ether, methyl ethyl ether, nitromethane) are also within the scope of this invention as are liquid fuels derived from vegetable or mineral sources such as corn, alfalfa, shale and coal. Normally liquid fuels which are mixtures of one or more hydrocarbonaceous fuels and one or more nonhydrocarbonaceous materials are also contemplated. Examples of such mixtures are combinations of gasoline and ethanol, and diesel fuel and ether. Particularly preferred is gasoline, that is, a mixture of hydrocarbons having an ASTM boiling point of about 60 °C at the 10 percent distillation point to about 205 °C at the 90 percent distillation point.

Generally, these fuel compositions contain an amount of the composition of this invention sufficient to impart friction modification and/or deposit softening properties to the fuel; usually this amount is bout 0.001 to about 5 percent (based on the weight of the final composition), preferably 0.001 percent to 1.0 percent.

The fuel compositions of this invention can contain, in addition to the compositions of this invention, other additives which are well known to those of skill in the art. These can include antiknock agents such as tetraalkyl lead compounds, lead scavengers such as halo-alkanes (e.g., ethylene dichloride and ethylene dibromide), deposit preventors or modifiers such as triaryl phosphates, dyes, cetane improvers, auxiliary antioxidants such as 2,6-ditertiary-butyl-4-methylphenol, rust inhibitors such as alkylated succinic acids and anhydrides, bacteriostatic agents, gum inhibitors, metal deactivators, demulsifiers, upper cylinder lubricants, anti-icing agents and the like.

In certain preferred fuel compositions of the present invention, the aforedescribed compositions are combined with an ashless dispersant in gasoline. Such ashless dispersants are preferably esters of a monoor polyol and a high molecular weight mono- or polycarboxylic acid acylating agent containing at least 30 carbon atoms in the acyl moiety. Such esters are well known to those of skill in the art. See, for example, French Patent No. 1,396,645, British Patent Nos. 981,850 and 1,055,337 and U.S. Patent Nos. 3,255,108; 3,311,558; 3,331,776; 3,346,354; 3,522,179; 3,579,450; 3,542,680; 3,381,022; 3,639,242; 3,697,428; 3,708,522; and British Patent Specification 1,306,529. These patents describe suitable esters and methods for their preparation. Generally, the weight ratio of the compositions of this invention to the aforesaid ashless dispersants is about 0.1 to about 10.0, preferably about 1 to about 10 parts of composition to 1 part ashless dispersant. In still another embodiment of this invention, the inventive additives are combined with Mannich condensation products formed from substituted phenols, aldehydes, polyamines, and substituted pyridines. Such condensation products are described in U.S. Patent Nos.

3,649,659; 3,558,743; 3,539,633; 3,704,308; and 3,725,277.

The compositions of this invention can be added directly to the fuel to form the fuel compositions of this invention or they can be diluted with a substantially inert, normally liquid organic solvent/diluent such as mineral oil, xylene, or a normally liquid fuel as described above, to form an additive concentrate which is then added to the fuel in sufficient amounts to form the inventive fuel composition described herein. These concentrates generally contain about 10 to 90 percent of the compositions of this invention and can contain in addition any of the above described conventional additives, particularly the aforedescribed ashless dispersants in the aforesaid proportions. The remainder of the concentrate is the solvent/diluent.

The lubricant fuel and additive concentrate compositions of this invention are exemplified by the following Table. All amounts other than those for mineral oil are exclusive of oil used as diluent.

| COMPONENT | PARTS BY WEIGHT | | |
|---|---|---|---|
| | I | II | III |
| Mineral Oil | 92.87 | 92.87 | 86.466 |
| Reaction Product of Malan-Styrene Copolymer with alcohol and heterocyclic amine | | | .124 |
| Hydrogenated styrene-isoprene non-dispersant viscosity improver | | | 8.93 |
| Polybutenyl succinic anhydride ethylene polyamine reaction product | 1.94 | 1.94 | 1.71 |
| Styrene-alkyl maleate copolymer | 1.79 | 1.79 | |
| Zinc isooctylphosphrodithioate | .78 | .78 | |
| Zinc methylamylphosphoridithioate | .67 | .67 | |
| Zinc salt of mixed isobutyl and primary amyl phosphorodithioates | | | .48 |
| Zinc salt of mixed isopropyl and primary amyl phosphorodithioates | | | .55 |

| | I | II | III |
|---|---|---|---|
| Basic calcium petroleum sulfonate | .52 | .52 | .25 |
| Basic sodium petroleum sulfonate | .42 | .42 | .30 |
| Basic magnesium petroleum sulfonate | | | .29 |
| Sulfurized Diels-Alder Adduct | | | .20 |
| Alkylated Aryl Amines | | | .09 |
| Oleamide/linoleamide mixture | | | .104 |
| Silicon anti-foamant | .005 | .005 | .006 |
| Product of Example 5 | 1.000 | | .50 |
| Product of Example 2 | | 1.000 | |

The fuel consumption of engines lubricated with compositions of this invention is measurably lower than that of engines lubricated with previously known lubricants. This can be shown by the Motored Engine Friction Horsepower Test, in which an engine is driven by a dynamometer at constant temperature as engine rpm. and torque are measured by a digital tachometer and a precision dial manometer, respectively. Friction horsepower, as calculated from these values, is roughly proportional to fuel consumed and thus decreases with improved fuel economy.

## Claims

**1.** A composition comprising one or more substituted succinimide derivatives represented by the formula:

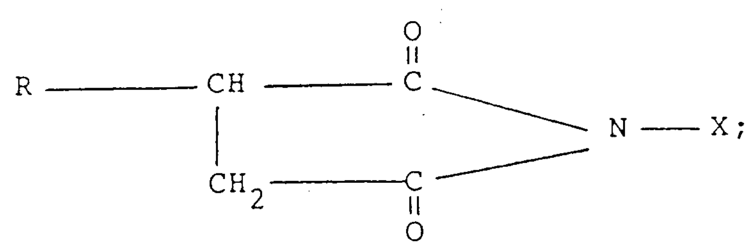

wherein R is an aliphatic hydrocarbon group, free from acetylenic unsaturation, containing from 10 up to 30 carbon atoms, in which at least 8 carbon atoms are in a straight-chain configuration, and X is magnesium, calcium, barium or zinc.

**2.** A process for the preparation of a composition according to claim 1 wherein one or more substituted succinimides represented by the formula:

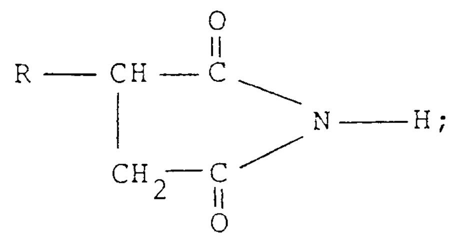

wherein R is an aliphatic hydrocarbon group, free from acetylenic unsaturation, containing from 10 up to 30 carbon atoms, in which at least 8 carbon atoms are in a straight-chain configuration, is reacted with at least one reactive metal, reactive metal derivative or mixtures thereof wherein the metal is magnesium, calcium, barium or zinc.

**3.** A process according to claim 2 wherein the reactive metal derivative is a metal salt.

**4.** An additive concentrate comprising a substantially inert, normally liquid organic diluent and from 10 percent up to 90 percent by weight of a composition according to claim 1 or a composition prepared by a process according to either of claims 2 and 3.

**5.** A lubricating composition a major amount of a lubricating oil and a minor amount of at least one composition according to claim 1 or a composition prepared by a process according to either of claims 2 and 3.

**6.** A fuel composition comprising a major amount of a normally liquid fuel and a minor amount of at least one composition according to claim 1 or a composition prepared by a process according to either of claims 2 and 3.

**7.** A method for reducing fuel consumption in an internal combustion engine which comprises lubricating said engine during operation with at least one lubricating composition comprising a composition according to claim 1 or a composition prepared by a process according to either of claims 2 and 3.

**8.** A process for the preparation of an additive concentrate which comprises admixing a substantially inert, normally liquid organic diluent and from 10 percent up to 90 percent by weight of a composition according to claim 1 or a composition prepared by a process according to either of claims 2 and 3.

**9.** A process for the preparation of a lubricating composition which comprises admixing a major amount of a lubricating oil and a minor amount of at least one composition according to claim 1 or a composition prepared by a process according to either of claims 2 and 3.

**10.** A process for the preparation of a fuel composition which comprises admixing a major amount of a normally liquid fuel and a minor amount of at least one composition according to claim 1 or a composition prepared by a process according to either of claims 2 and 3.

**Patentansprüche**

**1.** Zusammensetzung, umfassend ein oder mehrere substituierte Succinimidderivate der Formel:

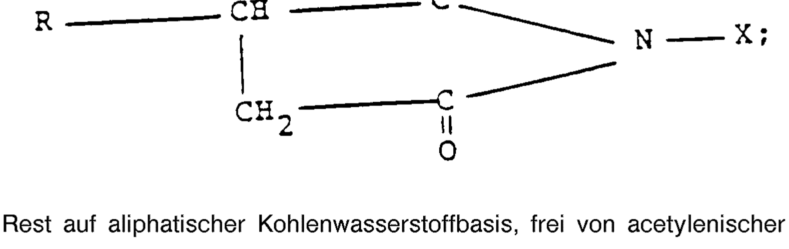

in der R einen Rest auf aliphatischer Kohlenwasserstoffbasis, frei von acetylenischer Ungesättigtheit,

mit 10 bis 30 Kohlenstoffatomen, in dem mindestens 8 Kohlenstoffatome in geradkettiger Konfiguration vorliegen, bedeutet und X ein Magnesium-, Calcium-, Barium- oder Zinkatom darstellt.

**2.** Verfahren zur Herstellung einer Zusammensetzung nach Anspruch 1, wobei ein oder mehrere substituierte Succinimide der Formel:

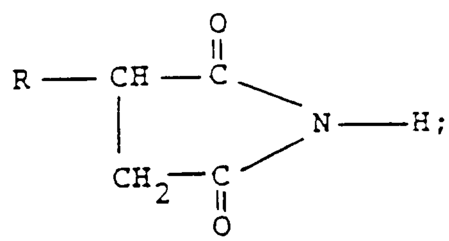

in der R einen auf aliphatischer Kohlenwasserstoffbasis, frei von acetylenischer Ungesättigtheit, mit 10 bis 30 Kohlenstoffatomen, in dem mindestens 8 Kohlenstoffatome in geradkettiger Konfiguration vorliegen, bedeutet, mit mindestens einem reaktiven Metall, reaktiven Metallderivat oder Gemischen davon umgesetzt wird, wobei das Metall Magnesium, Calcium, Barium oder Zink ist.

**3.** Verfahren nach Anspruch 2, wobei das reaktive Metallderivat ein Metallsalz ist.

**4.** Additivkonzentrat, umfassend ein im wesentlichen inertes, normalerweise flüssiges organisches Lösungsmittel und 10 bis 90 Gew.-% einer Zusammensetzung nach Anspruch 1 oder einer Zusammensetzung, gewonnen durch ein Verfahren nach einem der Ansprüche 2 und 3.

**5.** Schmiermittel, umfassend eine Hauptmenge eines Schmieröls und eine geringere Menge mindestens einer Zusammensetzung nach Anspruch 1 oder einer Zusammensetzung, gewonnen durch ein Verfahren nach einem der Ansprüche 2 und 3.

**6.** Treibstoffzusammensetzung, umfassend eine Hauptmenge eines normalerweise flüssigen Treibstoffs und eine geringere Menge mindestens einer Zusammensetzung nach Anspruch 1 oder einer Zusammensetzung, gewonnen durch ein Verfahren nach einem der Ansprüche 2 und 3.

**7.** Verfahren zur Verminderung des Treibstoffverbrauchs in Verbrennungsmotoren, umfassend die Schmierung des Motors während des Betriebes mit mindestens einem Schmiermittel, umfassend eine Zusammensetzung nach Anspruch 1 oder eine Zusammensetzung, gewonnen durch ein Verfahren nach einem der Ansprüche 2 und 3.

**8.** Verfahren zur Herstellung eines Additivkonzentrats, umfassend das Vermischen eines im wesentlichen inerten normalerweise flüssigen organischen Verdünnungsmittels mit 10 bis 90 Gew.-% einer Zusammensetzung nach Anspruch 1 oder einer Zusammensetzung, gewonnen durch ein Verfahren nach einem der Ansprüche 2 und 3.

**9.** Verfahren zur Herstellung eines Schmiermittels, umfassend das Vermischen einer Hauptmenge eines Schmieröls und einer geringeren Menge mindestens einer Zusammensetzung nach Anspruch 1 oder einer Zusammensetzung, gewonnen durch ein Verfahren nach einem der Ansprüche 2 und 3.

**10.** Verfahren zur Herstellung einer Treibstoffzusammensetzung, umfassend das Vermischen einer Hauptmenge eines normalerweise flüssigen Treibstoffs und einer geringeren Menge mindestens einer Zusammensetzung nach Anspruch 1 oder einer Zusammensetzung, gewonnen durch ein Verfahren nach einem der Ansprüche 2 und 3.

## Revendications

**1.** Une composition comportant un ou plus d'un dérivé de succinimide substitué, représenté par la formule :

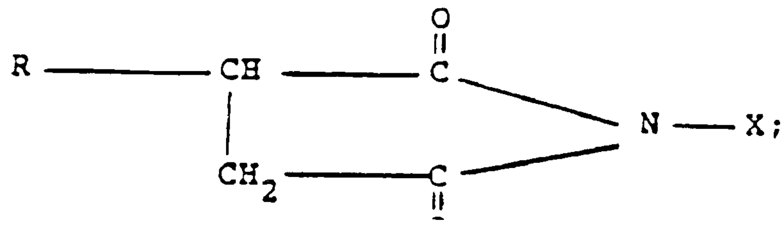

dans laquelle R est un groupe à base d'hydrocarbone aliphatique, dépourvu d'insaturation acétylénique renfermant de 10 jusqu'à 30 atomes de carbone, dans lequel au moins huit atomes de carbone présentent une configuration en chaîne droite et X est du magnésium, du calcium, du baryum ou du zinc.

2. Un procédé pour la préparation d'une composition selon la revendication 1, dans lequel un ou plus d'un succinimide substitué représenté par la formule :

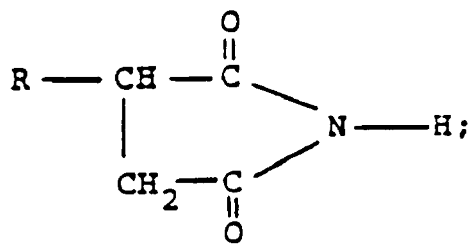

dans laquelle R est un groupe à base d'hydrocarbone, exempt d'insaturation acétylénique, renfermant de 8 jusqu'à 35 atomes de carbone, est mis à réagir avec au moins un métal réactif, un dérivé de métal réactif ou leurs mélanges, le métal étant du magnésium, du calcium, du baryum ou du zinc.

3. Un procédé selon la revendication 2, dans lequel le dérivé de métal réactif est un sel métallique.

4. Un produit concentré additif comportant un diluant organique, sensiblement inerte, liquide sous des conditions normales et de 10 % jusqu'à 90 % en poids d'une composition selon la revendication 1 ou bien une composition préparée par un procédé selon l'une quelconque des revendications 2 et 3.

5. Une composition lubrifiante comportant une quantité prépondérante d'une huile lubrifiante et une quantité mineure d'au moins une composition selon la revendication 1 ou bien une composition préparée par un procédé selon lune quelconque des revendications 2 et 3.

6. Une composition combustible comportant une quantité prépondérante d'un combustible liquide sous les conditions normales et une quantité mineure d'au moins une composition selon la revendication 1 ou bien une composition préparée par un procédé selon l'une quelconque des revendications 2 et 3.

7. Une méthode pour réduire la consommation de combustible dans un moteur à combustion interne, qui consiste à lubrifier ledit moteur pendant son fonctionnement avec au moins une composition lubrifiante comportant une composition selon la revendication 1 ou bien une composition préparée par un procédé selon l'une quelconque des revendications 2 et 3.

8. Un procédé pour la préparation d'un produit additif concentré qui consiste à mélanger un diluant organique, pratiquement inerte, liquide sous les conditions normales, et de 10 % jusqu'à 90 % en poids d'une composition selon la revendication 1 ou bien une composition préparée par un procédé selon l'une quelconque des revendications 2 et 3.

9. Un procédé pour la préparation d'une composition lubrifiante, selon lequel on mélange une quantité prépondérante d'une huile lubrifiante et une quantité mineure d'au moins une composition selon la revendication 1 ou bien une composition préparée par un procédé selon l'une quelconque des

revendications 2 et 3.

**10.** Un procédé pour la préparation d'une composition combustible selon lequel on mélange une quantité prépondérante d'un combustible liquide sous les conditions normales et une quantité mineure d'au moins une composition selon la revendication 1 ou bien une composition préparée par un procédé selon l'une quelconque des revendications 2 et 3.